(19) Europäisches Patentamt
European Patent Office
Office européen des brevets

(11) Veröffentlichungsnummer: **0 217 730**
**B1**

(12) **EUROPÄISCHE PATENTSCHRIFT**

(45) Veröffentlichungstag der Patentschrift: **12.12.90**

(51) Int. Cl.[5]: **C 07 D 457/12,** C 07 D 457/02
// A61K31/48

(21) Anmeldenummer: **86730117.8**

(22) Anmeldetag: **25.07.86**

# (54) Verfahren zur Herstellung von Ergolinyl-thioharnstoffen.

(30) Priorität: **06.08.85 DE 3528576**

(43) Veröffentlichungstag der Anmeldung:
**08.04.87 Patentblatt 87/15**

(45) Bekanntmachung des Hinweises auf die Patenterteilung:
**12.12.90 Patentblatt 90/50**

(84) Benannte Vertragsstaaten:
**AT BE CH DE FR GB IT LI LU NL SE**

(56) Entgegenhaltungen:
**EP-A-0 082 808**
**EP-A-0 126 968**
**AT-B- 278 853**
**DE-A-2 035 008**
**DE-B-1 119 843**

**Die Akte enthält technische Angaben, die nach dem Eingang der Anmeldung eingereicht wurden und die nicht in dieser Patentschrift enthalten sind.**

(73) Patentinhaber: **SCHERING AKTIENGESELLSCHAFT Berlin und Bergkamen**
**Müllerstrasse 170/178 Postfach 65 03 11**
**D-1000 Berlin 65 (DE)**

(72) Erfinder: **Biere, Helmut, Dr.**
**Zeltinger Strasse 15**
**D-1000 Berlin 28 (DE)**
Erfinder: **Haffer, Gregor, Dr.**
**Mörchinger Strasse 79**
**D-1000 Berlin 37 (DE)**

**EP 0 217 730 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die Erfindung betrifft ein neues Verfahren zur Herstellung von Ergolinyl-thioharnstoff-derivaten durch Austausch des Carbonylsauerstoffs von Ergolinyl-harnstoffderivaten gegen Schwefel.

Die Erfindung eignet sich besonders zur Herstellung von Ergolinyl-thioharnstoff-derivaten der allgemeinen Formel I

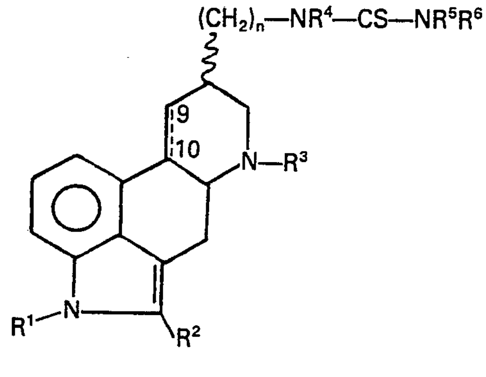

(I)

worin

$R^1$ Wasserstoff, niederes Alkyl oder Acyl,
$R^2$ Wasserstoff, Halogen oder eine niedere Alkylthiogruppe und
$R^3$ niederes Alkyl,
$R^4$, $R^5$, und $R^6$ gleich oder verschieden sind und jeweils Wasserstoff oder niederes Alkyl bedeuten,
n = 0, 1 oder 2 ist und
$C_9 \overset{---}{=} C_{10}$ eine Einfach- oder Dopplebindung darstellt.

Die erfindungsgemäß herstellbaren Thioharnstoffderivate der allgemeinen Formel I sind entweder selbst pharmakologisch wirksam (DE—OS 32 40 727, EP 82 808) oder können als Zwischenprodukte zur Herstellung von wertvollen Arzneimitteln verwendet werden.

Der Begriff niederes Alkyl in Formel I bedeutet $C_{1-4}$ Alkyl, wie z.B. Methyl, Ethyl, n-Propyl, iso-Propyl, n-Butyl, sek. Butyl, tert. Butyl, Isobutyl.

Unter Halogen ist Chlor, Brom oder Jod zu verstehen.

Die niedere-Alkylthiogruppe hat bevorzugt 1—2 Kohlenstoffatome wie z.B. die Methylthio- oder Ethylthio-gruppe.

Der Acylrest $R^1$ hat bis zu 4 Kohlenstoffatome und leitet sich beispielsweise von Ameisensäure, Essigsäure, Propionsäure oder Buttersäure ab.

Der Substituent in 8-Stellung kann α- oder β-ständig sein. Stellt $C_9 \overset{---}{=} C_{10}$ eine CC-Einfachbindung dar, so ist das Wasserstoffatom in 10-Stellung α-ständig.

Nach den bisher bekannten Verfahren werden zur Herstellung von Ergolin-thioharnstoffderivaten die schwer zugänglichen 8-Aminoergoline verwendet. Jedoch sind beispielsweise 8-Amine von 9,10-Didehydro-ergolinderivaten nur über den vielstufigen Curtius-Abbau zugänglich, hierbei entsteht ein 8 α, β-Stereoisomerengemisch, das mit zusätzlichem Arbeitsaufwand, der auch zu einem Ausbeuteverlust führt, aufgetrennt werden muß. [A. Hofmann, Helv. *30*, 44 (1947)].

Auch die 8-Amino-ergolinderivate mit einer 9,10-CC-Einfachbindung können nur über vielstufige Syntheseschritte in schlechten Ausbeuten hergestellt werden. (EP 48 695).

Die so erhaltenen 8-Ergolinylamine werden bislang zur Herstellung von Thioharnstoffderivaten entweder mit Isothiocyanaten oder 1,1′-Thiocarbonyl-diimidazol umgesetzt.

Setzt man Isothiocyanate ein, so gelangt man in einem Reaktionsschritt zu Thioharnstoffderivaten. Hiermit sind jedoch 1,1-Dialkylthioharnstoff, die pharmakologisch besonders wertvoll sind, nicht darstellbar.

Sollen, 1,1-Dialkylthioharnstoffderivative hergestellt werden, so läßt man 8-Ergolinylamine mit 1,1′-Thiocarbonyldiimidazol reagieren, und anschließend wird das erhaltene reaktive Zwischenprodukt mit einem primären oder sekundären Amin zu dem gewünschten Thioharnstoffderivat umgesetzt.

Für diese Darstellung muß also hochgiftiges und teures und daher industriell bedenkliches "Thiophosgen Reagenz" eingesetzt werden.

Aufgabe der vorliegenden Erfindung war es daher, ein Verfahren zur Herstellung von Ergolin-thioharnstoffderivaten zu entwickeln, das nicht die beschriebenen Nachteile besitzt, die den bekannten Verfahren anhaften, und das eine einfache und wirtschaftliche Herstellung der Ergolin-thioharnstoffderivate in guten Ausbeuten ermöglicht.

Zur Lösung dieser Aufgabe wurde gefunden, daß die leicht und in guten Ausbeuten zugänglichen Ergolin-8-yl-harnstoff-derivate (EP 21 206) unter milden Reaktionsbedingungen ohne Konfigurationsänderung und in guten Ausbeuten mit einem Thiolierungsmittel in die gewünschten Thioharnstoffderivate überführt werden können.

Das erfindungsgemäß Verfahren ist dadurch gekennzeichnet, daß man eine Verbindung der allgemeinen Formel II

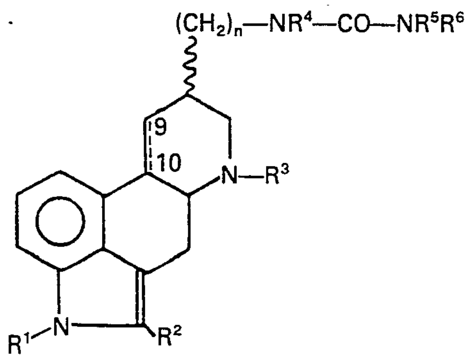

(II)

worin $R^1$, $R^2$, $R^3$, $R^4$, $R^5$, $R^6$ und $C_9 \overset{---}{=} C_{10}$ die obige Bedeutung haben, mit Phosphoroxychlorid und Alkalixanthogenaten, Alkali- oder Erdalkalisulfiden, Thioharnstoff oder Bunte-Salzen umsetzt.

Es tritt weder eine Chlorierung am Indol noch eine Konfigurationsänderung noch eine Reaktion an der Doppelbindung ein.

Hierzu wird das Ergolin-8-yl-harnstoffderivat mit Phosphoroxychlorid in ein reaktives Ergolinsalz überführt und dieses mit einem Thiolierungsreagenz zu den entsprechenden Ergolin-8-yl-thioharnstoffderivaten umgesetzt.

Die Umsetzung zum Ergolinsalz wird in einem inerten, vorzugsweise aprotischen Lösungsmittel vorgenommen, beispielsweise in chlorierten Kohlenwasserstoffen wie Dichlormethan, Dichloräthan, Ethern wie Diethylether, Tetrahydrofuran; Ketonen wie Aceton, Methylethylketon; Acetonitril u.a.

Die Reaktionstemperatur liegt zwische $-50°C$ bis $+80°C$, wobei Temperaturen von $-20°C$ bis Raumtemperatur als bevorzugt anzusehen sind. Phosphoroxychlorid wird mit einem 2—10 molaren überschuß, bevorzugt mit einem 2—4 molaren Überschuß, in die Reaktion eingesetzt.

Die so ernaltenden aktivierten Ergolin-harnstoffsalze können unter Feuchtigkeitsausschluß isoliert oder ohne Isolierung mit dem Thiolierungsmittel umgesetzt werden.

Als Thiolierungsmittel sind alle bekannten Thio-Nucleophile geeignet wie beispielsweise Alkali-Xanthogenate, Alkali- oder Erdalkalisulfide, Thioharnstoff, "Bunte-Salze" u.a., wobei Alkali-Xanthogenate wie Kalium-ethyl-xanthogenat, Kalium-methylxanthogenat und Alkalisulfide wie Natriumsulfid, Kaliumsulfid als bevorzugt anzusehen sind.

Zur Durchführung der Thiolierungsreaktion sind alle inerten Lösungsmittel geeignet sowohl die oben genannten als auch Dimethylformamid, Dimethylsulfoxid u.a. und auch protische Lösungsmittel wie Alkohole, z.B. Ethanol, Methanol, Propanol, u.a.

Die Reaktion läuft sowohl in homogener wie auch in heterogener Phase bei niederen Temperaturen rasch und vollständig zu den gewünschten Endprodukten ab.

Im allgemeinen wird die Thiolierung bei $-20°C$ bis Raumtemperatur vorgenommen, jedoch kann zur Beschleunigung und Vervollständigung der Reaktion in besonderen Fällen auch bei erhöhter Temperatur gearbeitet werden.

Die Umsetzung ist nach 1—5 Stunden beendet und wird im allgemeinen unter Schutzgasatmosphäre wie z.B. unter Argon oder Stickstoff durchgeführt.

Die Aufarbeitung der Endprodukte erfolgt nach den üblichen Verfahren gegebenenfalls auch durch chromatographische Reinigung.

Die nachfolgenden Beispiele sollen das erfindungsgemäße Verfahren erläutern:

Das bisher nicht beschriebene Ausgangsmaterial wurde auf folgende Weise hergestellt:

1,1-Diethyl-3-(1,6-di-n-propyl-8α-ergolinyl)-harnstoff.

2 g 1,1-Diethyl-3-(6-n-propyl-8α-ergolinyl)-harnstoff, 2,7 g gepulvertes KOH, 217 mg Tetrabutylammoniumhydrogensulfat und 5,5 ml n-Propyljodid werden in 100 ml abs. Tetrahydrofuran unter Stickstoff 5 Stunden bei Raumtemperatur gerührt. Nach Zusatz von 50 ml $H_2O$ wird mit Ethylacetat extrahiert, nachgewaschen und getrocknet. Mancerhält 1,8 g (80% d.Th.) als Öl. $[\alpha]_D = +2{,}6°C$ (c = 0,5 $CHCl_3$).

1,1-Diethyl-3-(1-ethyl-9,10-didehydro-6-methyl-8α-ergolinyl)-harnstoff.

Analog oben aus Lisurid durch $N^1$-Alkylierung mit Ethyljodid in Gegenwart von KOH und Tetrabutylammonium-hydrogensulfat in quantitativer Ausbeute.

## Beispiel 1

1,1-Diethyl-3-(6-methyl-8α-ergolinyl)-thioharnstoff

a) Eine Lösung von 1,4 g Phosphoroxychlorid in 10 ml abs. Dichlormethan wird auf $-20°C$ abgekühlt und unter Stickstoff mit 1.0 g 1,1-Diethyl-3-(6-methyl-8α-ergolinyl)-harnstoff versetzt. Das Reaktionsgemisch wird 6 h bei $-20°C$, dann über Nacht bei Raumtemperatur gerührt. Nach Abdestill. (i.Vak.) von Dichlormethan und überschüssigem $POCl_3$ wird der Rückstand unter Feuchtigkeitsausschluß mit Diethylether verrührt und die kristalline Masse rasch abgesaugt sowie über $P_2O_5$ getrocknet.

Das hydroskopische Kristallisat wird in 10 ml abs. Acetonitril suspendiert, auf $-10°C$ gekühlt und mit

1.5 g Kaliumethylxanthogenat versetzt. Nach Rühren der Suspension über Nacht unter Stickstoff und Feuchtigsausschluß bei Raumtemperatur wird das Acetonitril abdestilliert, der Rückstand mit $NaHCO_3$-Lösung und Ethylacetat verrührt, die organische Phase abgetrennt und i.Vak. eingeengt. Der Rückstand wird aus Dichlormethan/Diisopropylether kristallisiert und ergibt 0.9 g (84.2%) Thioharnstoff von Schmelzpunkt 205—207°C. $[\alpha]_D$ = +38° (c = 0,5 $CHCl_3$).

b) Das nach Beispiel 1a) dargestellte aktivierte Harnstoff-Derivat wird mit Kaliummethylxanthogenat in Dimethylformamid unter analogen Bedingungen mit Thioharnstoff umgesetzt. Ausbeute 81%, Schmp. 206°C.

c) Das nach Beispiel 1a) dargestellte aktivierte Harnstoff-Derivat wird mit Natriumsulfid in Acetonitril zum Thioharnstoff umgesetzt. Ausbeute 76%, Schmp. 207°C.

d) Das nach Beispiel 1a) dargestellte aktivierte Harnstoff-Derivat wird mit Kaliumethylxanthogenat in Ethanol (abs.) bei −20°C 30 Minuten gerührt, anschließend 2 Stunden bei Raumtemperatur gehalten. Anschliessend wird unter Eiskühlung tropfenweise mit 4 N KOH versetzt und mit Wasser gefällt. Ausbeute 81.9%.

Analog 1a) werden dargestellt:

1,1-Diethyl-3-(9,10-didehydro-6-methyl-8α-ergolinyl)-thioharnstoff
$[\alpha]_D$ = +395° (c = 0,5 Pyridin) aus 1,1-Diethyl-3-(9,10-didehyro-6-methyl-8α-ergolinyl)-harnstoff.

3-(2-Brom-6-methyl-8α-ergolinyl)-1,1-diethyl-thioharnstoff
$[\alpha]_D$ = +46° (c = 0,5 MeOH), aus 3-(2-Brom-6-methyl-8α-ergolinyl)-1,1-diethylharnstoff.

1,1-Diethyl-3-(1-ethyl-9,10-didehydro-6-methyl-8α-ergolinyl)-thioharnstoff
$[\alpha]_D$ = +354° (c = 0,5 $CHCl_3$) aus 1,1-Diethyl-3-(1-ethyl-9,10-didehydro-6-methyl-8α-ergolinyl)-harnstoff.

## Beispiel 2

1,1-Diethyl-3-(2-jod-6-methyl-8α-ergolinyl)-thioharnstoff

Eine auf −20°C gekühlte Lösung von 0.17 g Phosphoroxychlorid in 8 ml Dichlormethan wird unter Stickstoffatmosphäre mit 0.17 g 1,1-Diethyl-3-(2-jod-6-methyl-8α-ergolinyl)-harnstoff versetzt und 5 h bei dieser Temperatur, anschließend über Nacht bei Raumtemperatur gerührt. Nach Abdestillieren des Lösungsmittels wird der Rückstand in Diethylether verrührt und das Lösungsmittel erneut i.Vak. abdestilliert. Der trockene Rückstand wird ohne Isolierung mit 5 ml Acetonitril (abs.) versetzt und unter Kühlung auf −10°C mit 0.18 mg Kaliumethylxanthogenat 4 h unter Stickstoff gerührt, anschließend über Nacht bei Raumtemperatur gerührt. Nach Aufarbeitung — wie in Beispiel 1 — wird das Rohprodukt über Kieselgel mit Dichlormethan aufgereinigt und aus Pentan/Diethylether kristallisiert. Ausbeute 116 mg (64%) von Schmp. 198°C; $[\alpha]_D$ = +59° (c = 0,5, $CHCl_3$).

Analog werden die folgenden Thioharnstoff dargestellt:

3-(2-Brom-9,10-didehydro-6-methyl-8α-ergolinyl)-1,1-diethylthioharnstoff
$[\alpha]_D$ = +385.2° (c = 0,5 Pyridin) aus 3-(2-Brom-9,10-didehydro-6-methyl-8α-ergolinyl)-1,1-diethylharnstoff.

1,1-Diethyl-3-(1,6-di-n-propyl-8α-ergolinyl)-thioharnstoff
$[\alpha]_D$ = +39,4° (c = 0,5 $CHCl_3$) aus 1,1-Diethyl-3-(1,6-di-n-propyl-8α-ergolinyl)-harnstoff.

1,1-Diethyl-3-(1-ethyl-6-methyl-8α-ergolinyl)-thioharnstoff
$[\alpha]_D$ = +28° (c = 0,5 $CHCl_3$) aus 1,1-Diethyl-3-(1-ethyl-6-methyl-8α-ergolinyl)-harnstoff.

1,1-Diethyl-3-(6-methyl-2-methylthio-8α-ergolinyl)-thioharnstoff
$[\alpha]_D$ = +55° (c = 0,5 $CHCl_3$), aus 1,1-Diethyl-3-(6-methyl-2-methylthio-8α-ergolinyl)-harnstoff.

1,1-Diethyl-3-(6-methyl-8α-ergolinylmethyl)-thioharnstoff
$[\alpha]_D$ = −28° (c = 0,5 Pyridin), aus 1,1-Diethyl-3-(6-methyl-8α-ergolinylmethyl)-harnstoff.

3-(9,10-Didehydro-6-methyl-8α-ergolinylmethyl)-1,1-diethyl-thioharnstoff
$[\alpha]_D$ = +264° (c = 0,5 Pyridin), aus 3-(9,10-Didehydro-6-methyl-8α-ergolinylmethyl)-harnstoff.

1-(9,10-Didehydro-6-methyl-8α-ergolinyl)-thioharnstoff
$[\alpha]_D$ = +398° (c = 0,25 Pyridin) aus 1-(9,10-Didehydro-6-methyl-8α-ergolinyl)-harnstoff.

3-(9,10-Didehydro-6-methyl-8α-ergolinyl)-1,1-diethyl-thioharnstoff
$[\alpha]_D$ = +185° (c = 0,5 Pyridin) aus 3-(9,10-Didehydro-6-methyl-8β-ergolinyl)-1,1-diethylharnstoff.

1-Ethyl-3-(9,10-didehydro-6-methyl-8α-ergolinyl)-thioharnstoff
$[\alpha]_D$ = +406° (c = 0,5 $CHCl_3$) aus 1-Ethyl-3-(9,10-didehydro-6-methyl-8α-ergolinyl)-harnstoff.

**Patentanspruch**

Verfahren zur Herstellung von Ergolinyl-thioharnstoffderivaten der allgemeinen Formel I

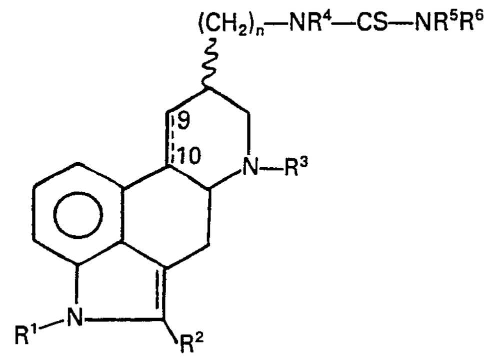

(I)

worin
$R^1$ Wasserstoff, niederes Alkyl oder Acyl,
$R^2$ Wasserstoff, Halogen oder eine niedere Alkylthiogruppe und
$R^3$ niederes Alkyl,
$R^4$, $R^5$, und $R^6$ gleich oder verschieden sind und jeweils Wasserstoff oder niederes Alkyl bedeuten,
$n = 0$, 1 oder 2 ist und
$C_9 \stackrel{---}{---} C_{10}$ eine Einfach- oder Doppelbindung darstellt,
dadurch gekennzeichnet, daß man eine Verbindung der allgemeinen Formel II

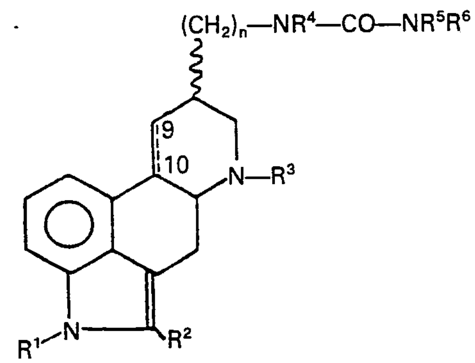

(II)

worin $R^1$, $R^2$, $R^3$, $R^4$, $R^5$, $R^6$, und $C_9 \stackrel{---}{---} C_{10}$ die obige Bedeutung haben, mit Phosphoroxychlorid und Alkalixanthogenaten, Alkali- oder Erdalkalisulfiden, Thioharnstoff oder Bunte-Salzen umsetzt.

**Revendication**

Procédé pour préparer des (ergolinyl)-thio-urées répondant à la formule générale I:

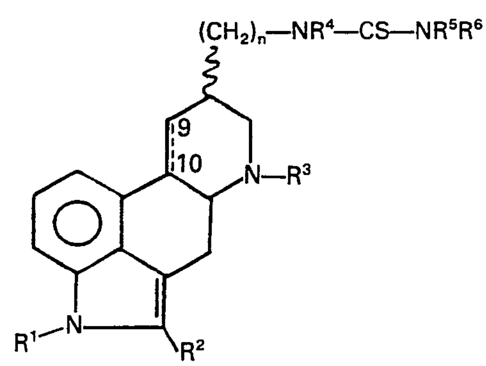

(I)

dans laquelle
$R^1$ représente l'hydrogène, un alkyle inférieur ou un acyle,
$R^2$ représente l'hydrogène, un halogène ou un alkylthio inférieur,
$R^3$ représente un alkyle inférieur,

$R^4$, $R^5$ et $R^6$ représente chacun, indépendamment l'un de l'autre, l'hydrogène ou un alkyle inférieur,
n est égal à 0, à 1 ou à 2 et
$C_9 \overset{---}{} C_{10}$ représente une liaison simple ou double, procédé caractérisé en ce qu'on fait réagir un composé répondant à la formule générale II

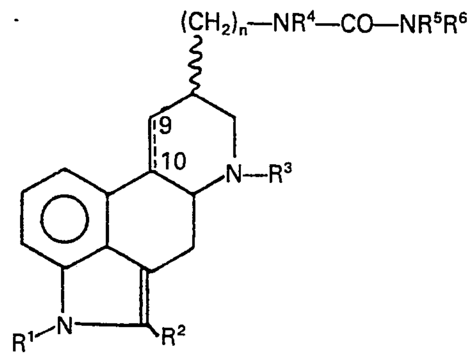

(II)

dans laquelle $R^1$, $R^2$, $R^3$, $R^4$, $R^5$, $R^6$ et $C_9 \overset{---}{} C_{10}$ ont les significations indiquées ci-dessus, avec l'oxychlorure de phosphore et des xanthogénates de métaux alacalins, des sulfures de métaux alcalins ou de métaux alcalino-terreux, la thio-urée ou des sels de Bunte.

**Claim**

Process for the preparation of ergolinyl-thiourea derivatives of the general formula I

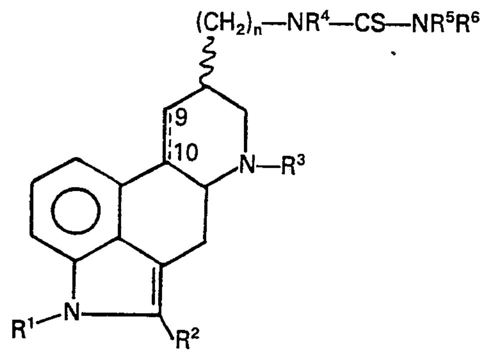

(I)

in which
$R^1$ represents hydrogen, lower alkyl or acyl,
$R^2$ represents hydrogen, halogen or a lower alkylthio group and
$R^3$ represents lower alkyl,
$R^4$, $R^5$ and $R^6$ are the same or different and each represents hydrogen or lower alkyl,
n = 0, 1 or 2 and
$C_9 \overset{---}{} C_{10}$ represents a single or double bond,
characterized in that a compound of the general formula II

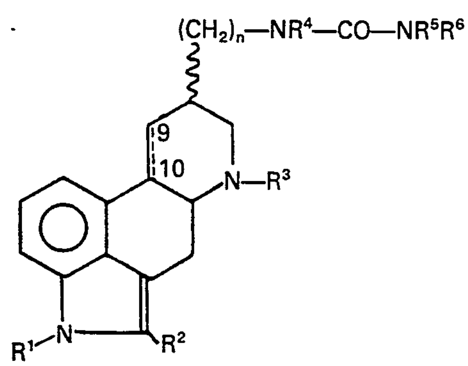

(II)

in which $R^1$, $R^2$, $R^3$, $R^4$, $R^5$, $R^6$ and $C_9 \overset{---}{} C_{10}$ have the meanings given above, is reacted with phosphorus oxychloride and alkali xanthogenates, alkali or alkaline earth sulphides, thiourea or Bunte salts.